# EUROPEAN PATENT APPLICATION

(11) **EP 3 372 980 A1**
(43) Date of publication of application: **12.09.2018**
(21) Application number: 15907748.6
(22) Date of filing: 05.11.2015
(51) Int. Cl.: G01N 1/04, G01N 33/50

(54) **STRATUM CORNEUM COLLECTION TOOL AND STRATUM CORNEUM COLLECTION/DETECTION KIT**

(71) Applicant: Shiseido Company Ltd., Chuo-ku Tokyo 104-0061 (JP)
(72) Inventor: TAKATA, Motoki, Yokohama-shi Kanagawa 224-8558 (JP); NASU, Mieko, Yokohama-shi Kanagawa 224-8558 (JP); TORII, Akihito, Yokohama-shi Kanagawa 224-8558 (JP); NAGOSHI, Masahiko, Yokohama-shi Kanagawa 224-8558 (JP)
(74) Representative: Scholz, Volker
(86) International application number: PCT/JP2015/005555
(87) International publication number: WO 2017/077562

(57) **Abstract**

[Objective]

To obtain a stratum corneum colleting/detecting kit capable of detecting and measuring a specific component in stratum corneum of skin with high precision.

[Constitution]

A kit is constituted by: a stratum corneum collecting device (10) formed by a rod shaped portion (11) having a grip portion (12) to be gripped by a user at one end, a columnar portion (15) which is continuous with the other end of the rod shaped portion (11), and an adhesive layer (16) formed on a circumferential surface of the columnar portion (15); and a solvent container (20), a solvent container, which is a bottomed cylindrical container having an opening in an upper portion thereof, containing a solvent for extracting a component from stratum corneum collected by the adhesive layer (16), the solvent container receiving the columnar portion (15) of the stratum corneum device through the opening so as to enable the adhesive layer (16) to be immersed in the solvent. A lid (13, 14) for closing the opening of the solvent container (20) after the adhesive layer (16) is immersed in the solvent, and a solvent outlet (13a) formed in the lid (13, 14) to be capable of maintaining a closed state, which is opened to take the solvent (S) out from the interior of the solvent container (20), are provided.

## Description

### [Technical Field]

The present invention is related to a stratum corneum collecting device for collecting the stratum corneum of skin.

The present invention further relates to a stratum corneum collecting/detecting kit for detecting a specific component from the stratum corneum of the skin.

### [Background Art]

Conventionally, as disclosed in, for example, Patent Documents 1, 2, and 3, detecting a specific component is detected from the stratum corneum of the skin, and determining the condition of the skin, namely a state of skin disorder, a parakeratosis state, etc. on a biochemical level based on the measured amount of the component, has been proposed. Skin conditions which are determined in this manner include skin disorders such as aging of skin caused by ultraviolet irradiation, parakeratosis caused by dry skin due to a reduction in skin moisturizing power due to a reduction in skin barrier function, parakeratosis caused by psoriasis or atopic dermatitis, and a skin disorder caused by hay fever, for example.

More specifically, Patent Documents 1 and 2 disclose quantitatively or qualitatively measuring squamous cell carcinoma antigens (hereinafter referred to as "SCCA") of stratum corneum cells, more specifically SCCA-1 and/or SCCA-2, and particularly SCCA-1 to determine the degree of sensitivity, responsiveness, and tenderness of the skin.

Patent Document 3 also discloses that skin diseases and sensitive skin can be examined by measuring the amount of nerve growth factors and/or neurotrophic factors 4 in a stratum corneum sample.

In order to determine the condition of the skin as described above, it is necessary to first obtain a stratum corneum sample. Patent Documents 1 through 3 disclose collecting stratum corneum samples by the so-called tape stripping method. The tape stripping method is a method that attaches a piece of adhesive tape on the surface layer of skin and then peels the piece of adhesive tape off, to collect a stratum corneum sample which is adhered to the peeled piece of adhesive tape.

### [Background Art Documents]

### [Patent Documents]

[Patent Document 1]
   Japanese Patent No. 5059600
[Patent Document 2]
   Japanese Unexamined Patent Publication No. 2007-279024
[Patent Document 3]
   Japanese Unexamined Patent Publication No. 2004-028698

### [Summary of the Invention]

### [Technical Problem]

The aforementioned tape stripping method can be conveniently executed. However, there remains room for improvement in terms of accuracy of detecting specific components of the stratum corneum. That is, the piece of tape which peels and holds the stratum corneum of the skin is immersed in a solvent which extracts a specific component of the stratum corneum. For this purpose, it is necessary to store a certain amount of solvent in a container. When extracting a specific component of the stratum corneum in a relatively large amount of solvent as described above, the concentration of the specific component in the solvent becomes comparatively low, and therefore the precision of detection for the specific component is likely to deteriorate.

The present invention has been developed in view of the foregoing circumstances. It is an object of the present invention to provide a stratum corneum collecting/detecting kit which is capable of detecting and measuring a specific component of the stratum corneum with high precision.

It is another object of the present invention to provide a stratum corneum collecting device that constitutes such a stratum corneum collecting/detecting kit.

### [Solution to Problem]

The stratum corneum collecting/detecting kit according to the present invention is characterized by comprising:
a stratum corneum collecting device comprising a rod shaped portion having a grip portion at one end to be gripped by a user, a columnar portion which is continuous with the other end of the rod shaped portion, and an adhesive layer formed on a circumferential surface of the columnar portion;
a solvent container, which is a bottomed cylindrical container having an opening in an upper portion thereof, containing a solvent for extracting a component from stratum corneum collected by the adhesive layer, the solvent container receiving the columnar portion of the stratum corneum collecting device through the opening so as to enable the adhesive layer to be immersed in the solvent; and
a lid for closing the opening of the solvent container after the adhesive layer is immersed in the solvent.

Here, in the stratum corneum collecting/detecting kit according to the present invention, it is desirable for the solvent container or the lid to be provided with a solvent outlet capable of maintaining a closed state, which is openable to take the solvent out from the interior to the exterior of the solvent container.

In addition, in the stratum corneum collecting/detecting kit according to the present invention, it is desirable for a cross sectional area of the internal space of the solvent container in a plane perpendicular to a cylinder axis direction to be smaller at the portion that receives the columnar portion than at the portion where the opening is formed.

In the stratum corneum collecting/detecting kit according to the present invention, it is desirable for the amount of the solvent to be set such that the liquid level of the solvent in the solvent container is located in the vicinity of the upper end of the adhesive layer which is immersed in the solvent.

In the stratum corneum collecting/detecting kit according to the present invention, it is desirable for the grip portion to also function as the lid.

Further, in the stratum corneum collecting/detecting kit according to the present invention, it is desirable for a guide means for positioning the stratum corneum collecting device which is inserted in the solvent container such that the adhesive layer does not come in contact with an inner wall of the solvent container to be provided.

Meanwhile, a stratum corneum collecting device according to the present invention is characterized by comprising:
a rod shaped portion having a grip portion at one end to be gripped by a user;
a columnar portion which is continuous with the other end of the rod shaped portion; and
an adhesive layer formed on a circumferential surface of the columnar portion.

### [Advantageous Effects of Invention]

The stratum corneum collecting/detecting kit of the present invention is configured so that the adhesive layer formed on the circumferential surface of the columnar portion is immersed in a solvent contained in the solvent container which is a cylindrical container. Therefore, it is possible to decrease the amount of the solvent to a minimum that will enable the solvent and the adhesive layer to contact with each other. That is, the amount of the solvent can be set to be that which thinly surrounds the exterior of the adhesive layer formed on the peripheral surface of the columnar portion. By suppressing the amount of solvent in the solvent container in this manner, the specific component of the stratum corneum, which is the target of detection and measurement, is extracted into the solvent at a relatively high concentration. Accordingly, detection and measurement of the specific component can be performed with high precision.

### [Brief Description of the Drawings]

[Figure 1] A perspective view that illustrates a stratum corneum collecting/detecting kit according to a first embodiment of the present invention.
[Figure 2] A schematic diagram for explaining a method of use of the stratum corneum collecting/detecting kit illustrated in Figure 1.
[Figure 3] A schematic diagram for explaining the method of use of the stratum corneum collecting/detecting kit illustrated in Figure 1.
[Figure 4] A perspective view that illustrates a state of the stratum corneum collecting/detecting kit illustrated in Figure 1 during use.
[Figure 5] A perspective view that illustrates another state of the stratum corneum collecting/detecting kit illustrated in Figure 1 during use.
[Figure 6] A perspective view that illustrates a stratum corneum collecting/detecting kit according to a second embodiment of the present invention.
[Figure 7] A perspective view that illustrates a stratum corneum collecting/detecting kit according to a third embodiment of the present invention.
[Figure 8] A schematic diagram for explaining a method of use of the stratum corneum collecting/detecting kit illustrated in Figure 7.
[Figure 9] A perspective view that illustrates a state of the stratum corneum collecting/detecting kit illustrated in Figure 7 during use.
[Figure 10] A perspective view that illustrates another state of the stratum corneum collecting/detecting kit illustrated in Figure 7 during use.
[Figure 11] A perspective view that illustrates a stratum corneum collecting/detecting kit according to a fourth embodiment of the present invention.
[Figure 12] A schematic diagram for explaining a method of use of the stratum corneum collecting/detecting kit illustrated in Figure 11.
[Figure 13] A perspective view that illustrates a state of the stratum corneum collecting/detecting kit illustrated in Figure 11 during use.
[Figure 14] A perspective view that illustrates another state of the stratum corneum collecting/detecting kit illustrated in Figure 11 during use.
[Figure 15] A perspective view that illustrates a stratum corneum collecting/detecting kit according to a fifth embodiment of the present invention.
[Figure 16] A schematic diagram for explaining a method of use of the stratum corneum collecting/detecting kit illustrated in Figure 15.
[Figure 17] A perspective view that illustrates a state of the stratum corneum collecting/detecting kit illustrated in Figure 15 during use.
[Figure 18] A perspective view that illustrates another state of the stratum corneum collecting/detecting kit illustrated in Figure 15 during use.
[Figure 19] A perspective view that illustrates a stratum corneum collecting/detecting kit according to a sixth embodiment of the present invention.
[Figure 20] A perspective view that illustrates a portion of the stratum corneum collecting/detecting kit illustrated in Figure 19.
[Figure 21] A perspective view that illustrates a state of the stratum corneum collecting/detecting kit illustrated in Figure 19 during use.
[Figure 22] A perspective view that illustrates another state of the stratum corneum collecting/detecting kit illustrated in Figure 19 during use.
[Figure 23] A perspective view that illustrates still another state of the stratum corneum collecting/detecting kit illustrated in Figure 19 during use.

### [Description of Embodiments]

Hereinafter, embodiments of the present invention will be described in detail with reference to the attached drawings.

### « First Embodiment »

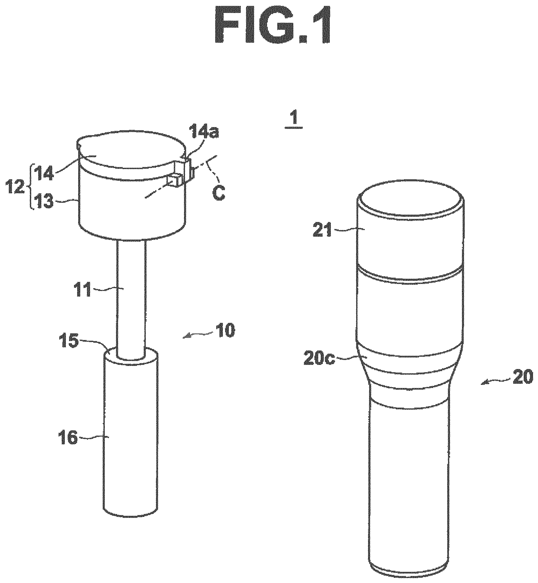

Figure 1 is a perspective view showing a stratum corneum collecting/detecting kit 1 according to a first embodiment of the present invention. The stratum corneum collecting/detecting kit 1 comprises a stratum corneum collecting device 10 for collecting stratum corneum from the skin of a subject, and a solvent container 20 that contains a solvent (extracting solution) that extracts a specific component within the collected stratum corneum. The stratum corneum collecting device 10 and the solvent container 20 are basically formed by molding a synthetic resin such as polystyrene, polypropylene, polyethylene, ABS resin, etc. except for an adhesive layer 16 which will be described later, but may also be formed from other materials.

The stratum corneum collecting device 10, as an example, has a cylindrical rod shaped portion 11, and a grip portion 12 to be gripped by a user is attached to one end thereof. The grip portion 12 is constituted by a plug portion 13 and a cap 14. The plug portion 13 is formed substantially in the shape of a bottomed cylinder, and the lower side, that is, the side closer to the rod shaped portion 11 is open while the opposite side (upper side) is closed as an upper base. The cap 14 is held by the plug portion 13 via a hinge portion 14a so as to be swingable about a swing axis C. The lower end portion of the cap 14 and the upper end portion of the plug portion 13 are configured such that, for example, a locking groove formed on one of the two and a locking protrusion (both not shown) formed on the other are capable of locking engagement. Thereby, a state in which the cap 14 covers the plug portion 13 can be maintained. Note that although the upper base portion of the plug portion 13 is covered and hidden by the cap 14 in Figure 1, the structure of the upper base portion of the plug portion 13 will be described in detail later. Further, a female threaded portion (not shown) for engaging with a male threaded portion 20b formed on the solvent container 20 is formed on the inner peripheral surface of the cylindrical plug portion 13. Further, a cylindrical columnar portion 15 having a diameter slightly larger than that of the rod shaped portion 11 is formed so as to be continuous with the other end of the rod shaped portion 11. An adhesive layer 16 is formed on the entire outer peripheral surface of the columnar portion 15.

The adhesive layer 16 is formed by applying an adhesive agent directly to the outer peripheral surface of the columnar portion 15 or by attaching a substrate in the form of a sheet, which is coated an adhesive agent, to the outer peripheral surface of the columnar portion 15. Specific examples of the adhesive agent include natural rubber, synthetic rubbers such as polyisobutylene rubber, polybutadiene rubber, silicone rubber, polyisoprene rubber and styrene-isoprene-styrene block copolymer rubber; and synthetic resins such as polypropylene resin, polystyrene resin, and polyacrylic acid ester copolymer resin. Rosin, hydrogenated rosin, and esters thereof, polyterpene resin, petroleum resin, etc. may also be employed as a tackifier. Specific examples of the substrate include paper; staple fiber; cotton; cloth; nonwoven cloth; films made of various resins such as polyester resin, urethane resin, polyethylene resin, ethylene-vinyl acetate copolymer, flexible polyvinyl chloride; aluminum foil, etc. These substrates may be employed singly or in combination. However, the adhesive agent, the tackifier, and the substrate are not limited to these examples.

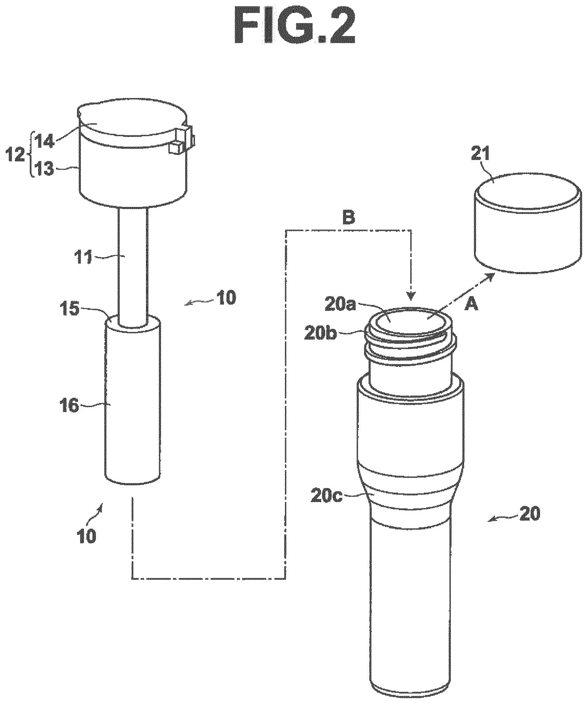

Meanwhile, as illustrated in greater detail in Figure 2, the solvent container 20 is an elongated, bottomed cylindrical container having an opening 20a at the upper portion thereof. The male threaded portion 20b is formed on the peripheral surface of the solvent container 20 in the vicinity of the upper end near the opening 20a. In addition, a portion close to the center position in the height direction of the solvent container 20 is a tapered portion 20c. A portion below the tapered portion 20c, that is, a portion for housing the columnar portion 15 of the stratum corneum collecting device 10 when the stratum corneum collecting device 10 is inserted into the solvent container 20 as will be described later, has an inner diameter which is smaller than an inner diameter of the portion above the portion 20c. In other words, considering cross sectional areas of the internal space of the solvent container 20 in planes perpendicular to the cylinder axis direction, the portion that receives the columnar portion 15 is smaller in cross sectional area than the portion where the opening 20 a is formed.

The solvent container 20 contains a solvent in the interior thereof. The solvent enables extraction of the specific component in stratum corneum which is collected by the adhesive layer 16 of the stratum corneum collecting device 10, as will be described later. A provisional plug 21 that prevents the solvent from flowing out is mounted on the solvent container 20. The provisional plug 21 is a substantially cylindrical bottomed member with its lower side opened while the upper side is closed as an upper base, in the same manner as the plug portion 13 of the stratum corneum collecting device 10. The provisional plug 21 is mounted on the solvent container 20 by threaded engagement with the female threaded portion (not shown) formed on an inner peripheral surface thereof onto the male threaded portion 20b.

Note that generally, the stratum corneum collecting device 10 and the solvent container 20 described above are provided for sale etc. in a packaging container, etc. (not shown).

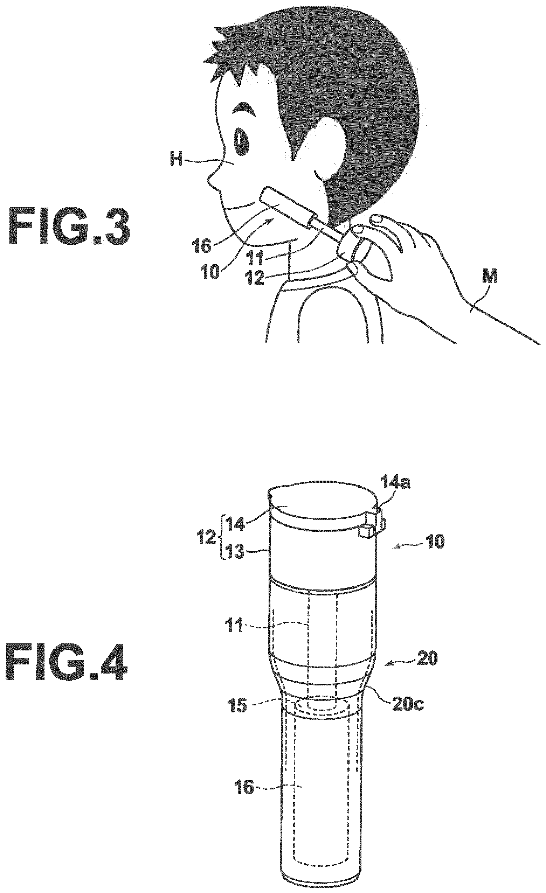

Next, a method of use of the stratum corneum collecting/detecting kit 1 of the present embodiment will be described. During an examination, the stratum corneum collecting device 10 is handled by the grip portion 12 being gripped by the hand M of a collector as illustrated in Figure 3, for example. Then, the adhesive layer 16 of the stratum corneum collecting device 10 is pressed against the skin of a subject H at a part to be examined of the subject H, for example, the skin of a cheek. By rotating the grip portion 12 in this state, that is, by rotating the adhesive layer 16, the stratum corneum is peeled and collected from the skin of the subject H over a wide range of the adhesive layer 16. The surface layer of the skin may be washed to remove sebum, dirt, makeup and the like prior to the stratum corneum being collected, but this is not necessary.

When the stratum corneum is collected, the provisional plug 21 is removed from the solvent container 20, as indicated by the dashed line A in Figure 2, and the opening 20a is exposed. The provisional plug 21 is removed by rotating the provisional plug 21 to release the threaded engagement with the male threaded portion 20b. Note that the provisional plug 21 may be removed prior to the collection of the stratum corneum.

The stratum corneum collecting device 10 which has collected and is holding the stratum corneum on the adhesive layer 16 is received within the solvent container 20 by inserting the columnar portion 15 through the opening 20a as indicated by the dashed line B in Figure 2. The aforementioned female threaded portion which is formed on the inner peripheral surface of the plug portion 13 establishes threaded engagement with the male threaded portion 20b which is formed on the solvent container 20 to integrate the stratum corneum collecting device 10 and the solvent container 20.

Figure 4 illustrates a state in which the stratum corneum collecting device 10 is housed within the solvent container 20. In this state, the adhesive layer 16 of the stratum corneum collecting device 10 is immersed in the solvent which is contained in the solvent container 20. In this state, the opening 20a of the solvent container 20 is closed by the plug portion 13 and the cap 14 of the stratum corneum collecting device 10. That is, the plug portion 13 and the cap 14 that constitute the grip portion 12 of the stratum corneum collecting device 10 also function as a lid that closes the opening 20a.

In addition, when the stratum corneum collecting device 10 and the solvent container 20 are integrated by establishing threaded engagement between the female threaded portion and the male threaded portion, the stratum corneum collecting device 10 is coaxial with the solvent container 20, and the adhesive layer 16 is held in the solvent container 20 such that it does not come into contact with an inner wall of the solvent container 20. That is, the plug portion 13 which has the female threaded portion and the male threaded portion 20b of the solvent container 20 function as a guide means that guides the stratum corneum collecting device 10 such that the adhesive layer 16 does not contact the inner wall of the solvent container 20 when the stratum corneum collecting device 10 is inserted into the solvent container 20.

It is preferable for the amount of the solvent contained in the solvent container 20 to be set to an amount that causes the liquid level of the solvent to be positioned in the vicinity of the upper end of the adhesive layer 16 in a state in which the stratum corneum collecting device 10 is housed in the solvent container 20. By setting the amount of the solvent in this manner, it becomes possible to suppress the amount of the solvent to the minimum amount necessary. In addition, if the stratum corneum collecting device 10 is adjusted such that the gap between the stratum corneum collecting devise 10 and the inner circumferential surface of the solvent container 20 is comparatively small, it will become possible to extract the specific component of the stratum corneum into the solvent at a high concentration, which is favorable. Here, the liquid level of the solvent may be higher or lower than the upper end of the adhesive layer 16. The amount of the solvent is specifically set to approximately 1 mL (milliliter), for example.

The specific component of the stratum corneum, which has been collected and is held on the adhesive layer 16, is extracted into the solvent, by housing the stratum corneum collecting device 10 in the solvent container 20 and immersing the adhesive layer 16 in the solvent which is maintained at a predetermined temperature for a predetermined amount of time. Note that Tris-buffer may be applied as the solvent, in the case that the expression of squamous cell carcinoma-associated antigens (SCCA) of the skin stratum corneum cells is measured to determine the sensitivity, the responsiveness, and the tenderness of the skin of the subject H as biochemical levels, as disclosed in Patent Documents 1 and 2, for example.

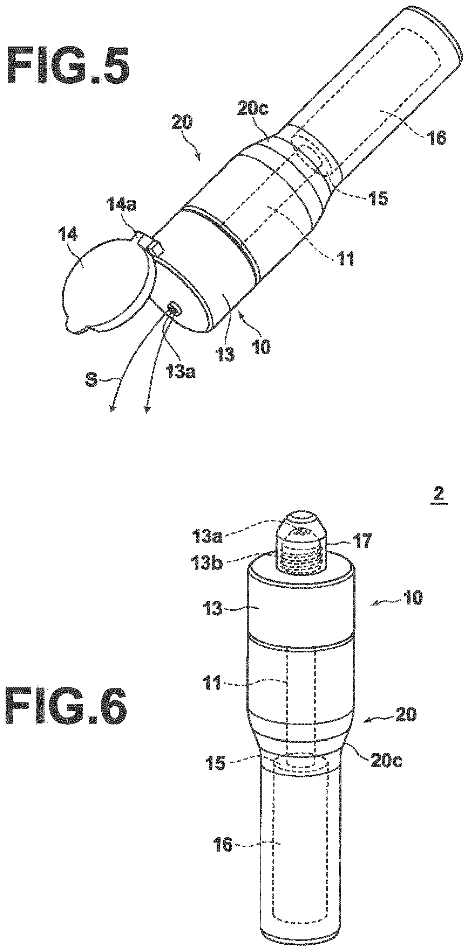

As described above, after the adhesive layer 16 is immersed in the solvent for a predetermined amount of time, the cap 14 is opened as illustrated in Figure 5, and the solvent S is taken out from the solvent container 20. That is, a solvent outlet 13a is formed at the bottom of the bottomed cylindrical plug 13, and the solvent S is caused to flow out from the solvent outlet 13a. Note that the solvent outlet 13a is capable of maintaining a closed state by attaching the cap 14 to the plug portion 13, and is open only when the cap 14 is opened as illustrated in Figure 5.

Here, the cap 14 and the solvent outlet 13a may not be provided in particular. The plug portion 13 may be removed from the solvent container 20, and the solvent S may be caused to flow out through the exposed opening 20a when the solvent S is to be taken out.

The solvent S which is taken out of the solvent container 20 is supplied to a measuring instrument that measures the extracted SCCA. The measurement of SCCA can be carried out quantitatively or qualitatively by applying any known method. Specific examples of immunoassay methods that utilize SCCA specific antibodies include the ELISA method which utilizes enzyme labels, the RIA method which utilizes radioactive labels, the immunoturbidimetric method, the western blotting method, the latex agglutination method, and the hemagglutination method. The immunoassay methods include competitive methods and sandwich methods.

In the stratum corneum collecting/detecting kit 1 of the present embodiment, as described above, the amount of the solvent in the solvent container 20 is suppressed to the minimum necessary amount. Therefore, the specific component of the stratum corneum, which is the target of detection and measurement, is extracted into the solvent at a comparatively high concentration. Accordingly, detection and measurement of the specific component can be performed with high precision.

In addition, in the stratum corneum collecting/detecting kit 1 of the present embodiment is in a state in which the portion that receives the columnar portion 15 has a smaller cross sectional area than the portion where the opening 20 a is formed in the solvent container 20 as described above. As a result, the volume of the portion that receives the columnar portion 15 is comparatively small, which is advantageous from the viewpoint of decreasing the amount of the solvent to be contained therein to the minimum necessary amount to immerse the adhesive layer 16. Meanwhile, the portion where the opening 20a is formed has a comparatively large diameter. Therefore, the outer diameter of the grip portion 12 to be combined therewith is also relatively large, facilitating holding of the grip portion 12.

In the stratum corneum collecting/detecting kit 1 of the present embodiment, the columnar portion 15 is larger in diameter than the rod shaped portion 11. However, these portions may be constituted by a single member having a uniform diameter. In addition, the rod shaped portion 11 and the columnar portion 15 may be formed in a columnar shape, for example, a quadrangular prism shape, a hexagonal prism shape, or formed in such shapes with hollow interiors (for example, a round tubular shape or a rectangular tubular shape), in addition to the cylindrical shape.

In order to cause the volume of the portion that receives the columnar portion 15 of the solvent container 20 to be comparatively smaller, the thickness of this portion may be increased instead of providing the tapered portion 20c described above. However, in such a case, the wall thickness of the portion that receives the columnar portion 15 of the solvent container 20 will become unnecessarily large, and the amount of synthetic resin utilized to form the solvent container 20 will increase, which is not favorable.

The stratum corneum collecting/detecting container and the kit according to the present invention are not limited to the SCCA measurement described above, and can be configured as a kit for measuring the amounts of factors related to any other skin conditions which are present. For example, the stratum corneum collecting/detecting kit may be configured as a kit for measuring the amount of nerve growth factors and/or neurotrophic factors 4 in the stratum corneum as described in Patent Document 3. In this case, a conventional physiological buffer solution may be applied as a solvent for extracting nerve growth factors or neurotrophic factors 4. Dulbecco's phosphate buffered saline solution or the like may be particularly favorably applied, but the solvent is not limited thereto.

### << Second Embodiment >>

Next, a second embodiment of the present invention will be described with reference to Figure 6. Note that in Figure 6, the same elements as those which have already been described are denoted by the same reference numerals, and descriptions thereof will be omitted unless particularly necessary (the same applies to all of the following embodiments).

Compared to the stratum corneum collecting/detecting kit 1 illustrated in Figures 1 and 2, a stratum corneum collecting/detecting kit 2 according to the second embodiment is different in the structure of the lid that closes the opening 20a of the solvent container 20. That is, in this stratum corneum collecting/detecting kit 2, a protruding solvent outflow portion 13b is formed at the upper base portion of the plug portion 13 of the stratum corneum collecting device 10. A solvent outlet 13a which is open at the upper end surface is provided within the solvent outflow portion 13b. A cap 17 is fitted on the solvent outflow portion 13b, and the solvent outlet 13a is closed by the cap 17. A male threaded portion is formed on the outer peripheral surface of the solvent outflow portion 13b, while a female threaded portion is formed on an inner peripheral surface of the cap 17. The cap 17 is fitted onto the solvent outflow portion 13b by establishing threaded engagement between the threaded portions.

In the case that the stratum corneum collecting/detecting kit 2 having the above configuration is employed as well, collection of the stratum corneum using the stratum corneum collecting device 10 and housing of the stratum corneum collecting device 10 in the solvent container 20 are carried out in the same manner as that in the case that the stratum corneum collecting/detecting kit 1of the first embodiment is employed. When taking solvent out from the solvent container 20 after immersing the adhesive layer 16 in the solvent contained in the solvent container 20, the cap 17 is rotated, the threaded engagement is disengaged, and the cap 17 is removed from the solvent outflow portion 13b. As a result, the solvent outlet 13a is opened and the solvent is taken out of the solvent container 20.

### << Third Embodiment >>

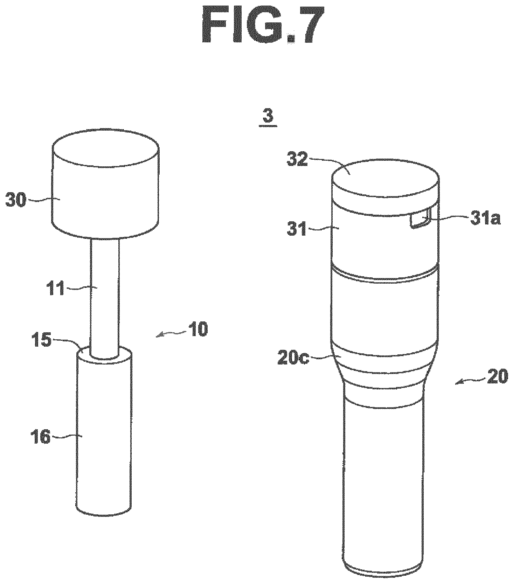

Next, a third embodiment of the present invention will be described with reference to Figures 7 through 10. Compared to the stratum corneum collecting/detecting kit 1 illustrated in Figures 1 and 2, a stratum corneum collecting/detecting kit 3 according to the third embodiment basically differs in the point that a lid that closes the opening 20a of the solvent container 20 after the adhesive layer 16 is immersed in the solvent is formed on the side of the solvent container 20.

That is, as illustrated in Figure 7, in the stratum corneum collecting device 10 that constitutes the stratum corneum collecting/detecting kit 3, a grip portion 30 which is formed as a bottomed cylinder is mounted on one end side of the rod shaped portion 11, that is, on the side opposite the columnar portion 15, in a manner similar to the plug portion 13 illustrated in Figure 1. During collection of stratum corneum using the stratum corneum collecting device 10, the grip portion 30 is gripped by a human hand to handle the stratum corneum collecting device 10.

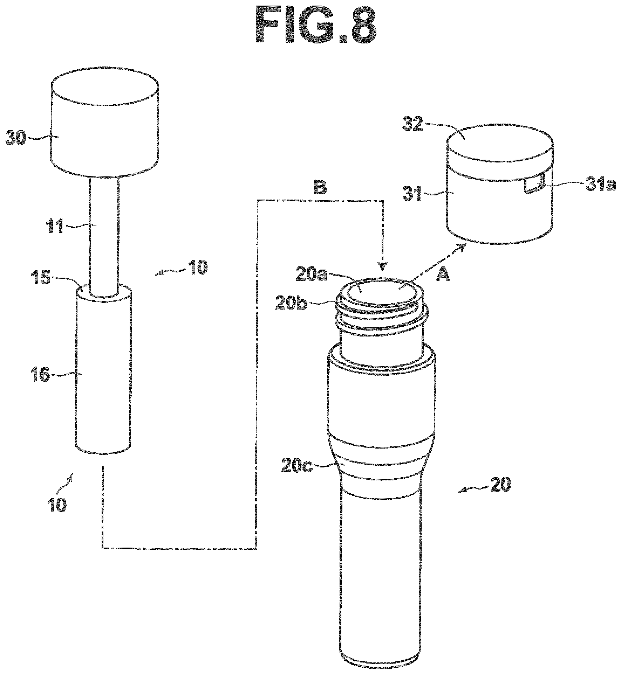

Meanwhile, a plug portion 31 which is formed as a bottomed cylinder is mounted on the solvent container 20 in a manner similar to the provisional plug 21 illustrated in Figure 1. The plug portion 31 is mounted on the upper portion of the solvent container by establishing threaded engagement between a female threaded portion (not shown) formed on the inner surface of the plug portion 31 and a male threaded portion 20b illustrated in Figure 8. A cap 32 that constitutes the lid with the plug portion 31 is mounted on the plug portion 31. The lower end portion of the cap 32 and the upper end portion of the plug portion 31 are configured such that a locking groove formed on one of the two and a locking protrusion (both not shown) formed on the other are capable of locking engagement, for example. Thereby, a state in which the cap 32 covers the plug portion 31 can be maintained. In addition, an unlocking portion 31a is provided at a portion of the outer peripheral surface of the plug portion 31. The unlocking portion 31a assists release of the locking engagement between the plug portion 31 and the cap 32 by flexing when pressed.

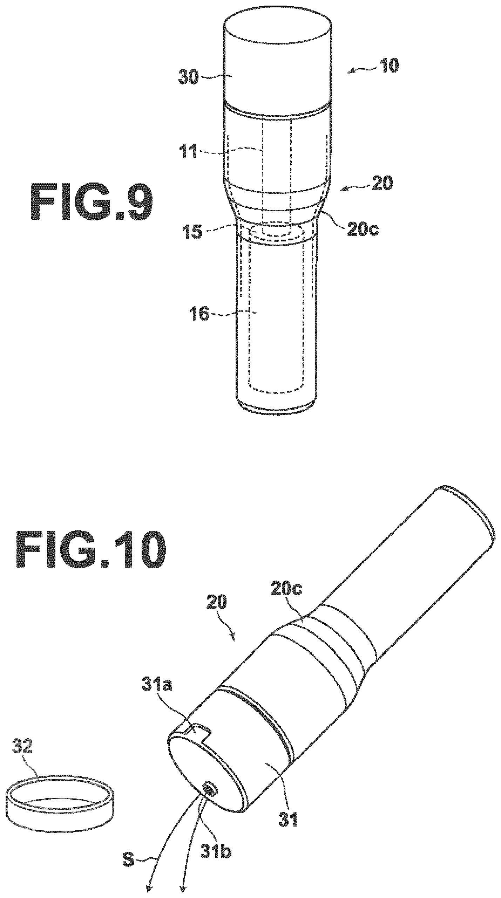

Hereinafter, a method of use of the stratum corneum collecting/detecting kit 3 of the present embodiment will be described. In the case that the stratum corneum collecting/detecting kit 3 is employed as well, the collection of the stratum corneum using the stratum corneum collecting device 10 and the housing of the stratum corneum collecting device 10 in the solvent container 20 are conducted in the same manner as the stratum corneum collecting/detecting kit 1 of the first embodiment. Note that prior to housing the stratum corneum collecting device 10 in the solvent container 20, the plug portion 31 is detached from the upper portion of the solvent container 20, as indicated by the dashed line A in Figure 8. The stratum corneum collecting device 10 is received in the solvent container 20 to be housed therein by the columnar portion 15 being inserted through the opening 20a, as indicated by the dashed line B in Figure 8. The state of the stratum corneum collecting/detecting kit 3 at this time is illustrated in Figure 9.

After immersing the adhesive layer 16 in the solvent in the solvent container 20 for a predetermined period of time to extract a specific component of the stratum corneum which has been collected and is held on the adhesive layer 16 into the solvent as described above, the stratum corneum collecting device 10 is removed from the solvent container 20. Then, the plug portion 31 is attached to the upper portion of the solvent container 20 again, and the opening 20a is closed.

Next, as illustrated in Figure 10, the locking engagement between the plug portion 31 and the cap 32 is released, and the cap 32 is removed from the plug portion 31. A solvent outlet 31b is open in the upper base portion of the plug portion 31. When the cap 32 is removed, the solvent outlet 31b is opened. In this state, when the solvent container 20 is brought into an orientation in which the upper portion thereof faces downward, the solvent S containing the extracted specific component of the stratum corneum is taken out from the solvent container 20 through the solvent outlet 31b.

Note that in the stratum corneum collecting/detecting kit of the present invention, the solvent container 20 may be formed of a material having a relatively high rigidity or may be formed of a material having a relatively low rigidity, which is flexible. In the case where the solvent container 20 is formed from a material having the latter properties, even if the solvent outlet 31b is considerably small and it is difficult for the solvent S to flow out therefrom, the outer circumferential surface of the solvent container 20 may be deformed by lightly pushing it inward, resulting in the solvent S flowing out smoothly therefrom.

### « Fourth Embodiment »

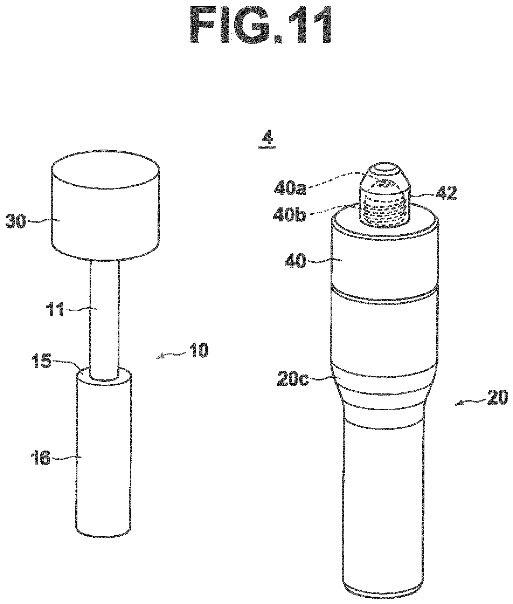

Next, a fourth embodiment of the present invention will be described with reference to Figures 11 through 14. Compared to the stratum corneum collecting/detecting kit 2 illustrated in Figure 6, the stratum corneum collecting/detecting kit 4 according to the fourth embodiment basically differs from the stratum corneum collecting/detecting kit 2 in the point that a lid that closes that opening 20a of the solvent container 20 after the adhesive layer 16 is immersed in the solvent is formed on the side of the solvent container 20.

That is, as illustrated in Figure 11, in the stratum corneum collecting device 10 that constitutes the stratum corneum collecting/detecting kit 4, a grip portion 30 which is formed as a bottomed cylinder is mounted on one end side of the rod shaped portion 11, that is, on the side opposite the columnar portion 15, in a manner similar to the plug portion 13 illustrated in Figure 1. During collection of stratum corneum using the stratum corneum collecting device 10, the grip portion 30 is gripped by a human hand to handle the stratum corneum collecting device 10.

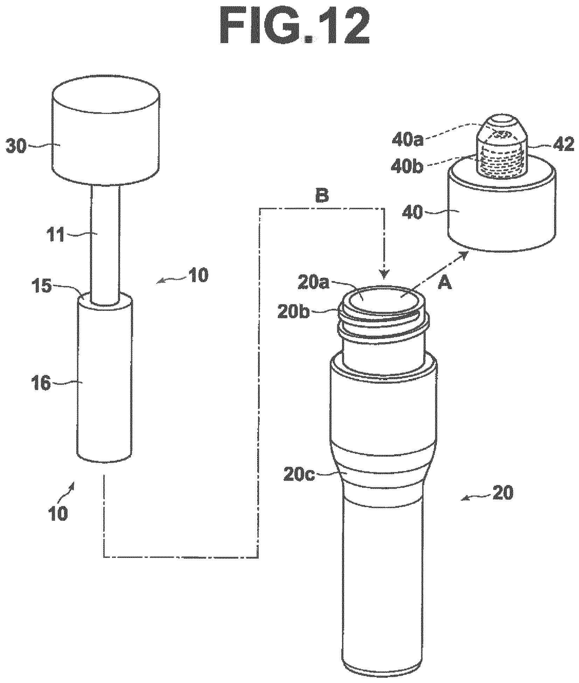

Meanwhile, a plug portion 40 which is formed as a bottomed cylinder is mounted on the solvent container 20. The plug portion 40 is mounted on the upper portion of the solvent container 20 by establishing threaded engagement between a female threaded portion (not shown) formed on the inner surface of the plug portion 40 and a male threaded portion 20b illustrated in Figure 12. A protruding solvent outflow portion 40b is formed at the upper base portion of the plug portion 40. A solvent outlet 40a which is open at the upper end surface is provided within the solvent outflow portion 40b. A cap 42 is fitted on the solvent outflow portion 40b, and the solvent outlet 40a is closed by the cap 42. Note that a male threaded portion is formed on the outer peripheral surface of the solvent outflow portion 40b, while a female threaded portion is formed on an inner peripheral surface of the cap 42. The cap 42 is fitted onto the solvent outflow portion 40b by establishing threaded engagement between the threaded portions.

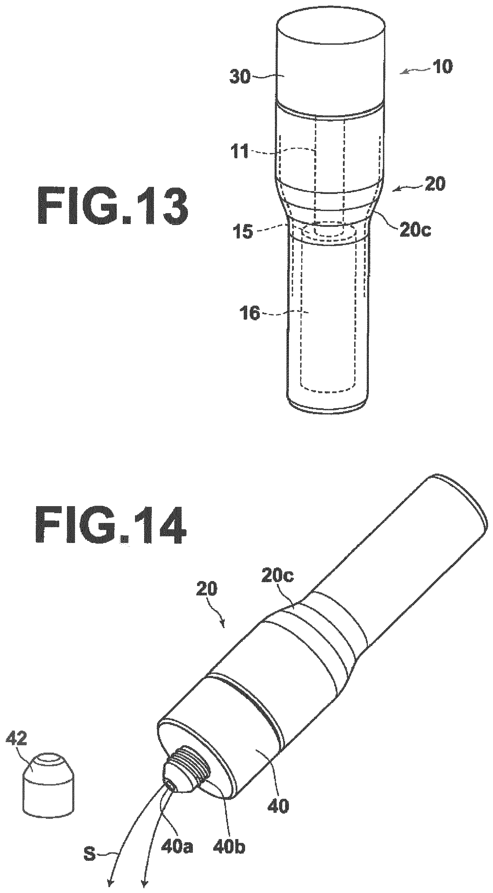

Hereinafter, a method of use of the stratum corneum collecting/detecting kit 4 of the present embodiment will be described. In the case that the stratum corneum collecting/detecting kit 4 is employed as well, the collection of the stratum corneum using the stratum corneum collecting device 10 and the housing of the stratum corneum collecting device 10 in the solvent container 20 are conducted in the same manner as the stratum corneum collecting/detecting kit 1 of the first embodiment. Note that prior to housing the stratum corneum collecting device 10 in the solvent container 20, the plug portion 40 is detached from the upper portion of the solvent container 20, as indicated by the dashed line A in Figure 12. The stratum corneum collecting device 10 is received in the solvent container 20 to be housed therein by the columnar portion 15 being inserted through the opening 20a, as indicated by the dashed line B in Figure 12. The state of the stratum corneum collecting/detecting kit 4 at this time is illustrated in Figure 13.

After immersing the adhesive layer 16 in the solvent in the solvent container 20 for a predetermined period of time to extract a specific component of the stratum corneum which has been collected and is held on the adhesive layer 16 into the solvent as described above, the stratum corneum collecting device 10 is removed from the solvent container 20. Then, the plug portion 40 is attached to the upper portion of the solvent container 20 again, and the opening 20a is closed.

Next, as illustrated in Figure 14, the threaded engagement between the solvent outflow portion 40b and the cap 42 is released, and the cap 42 is removed from the plug portion 40. When the cap 42 is removed, the solvent outlet 40a is in an open state. In this state, when the solvent container 20 is brought into an orientation in which the upper portion thereof faces downward, the solvent S containing the extracted specific component of the stratum corneum is taken out from the solvent container 20 through the solvent outlet 40a.

### << Fifth Embodiment >>

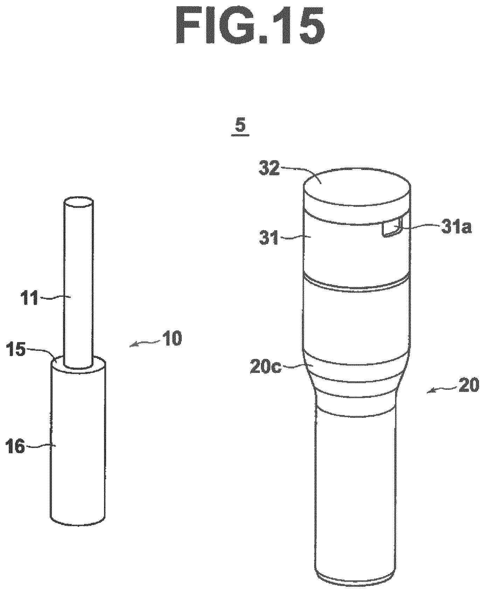

Next, a fifth embodiment of the present invention will be described with reference to Figures 15 through 18. Compared to the stratum corneum collecting/detecting kit 3 illustrated in Figures 7 through 10, a stratum corneum collecting/detecting kit 5 according to the fifth embodiment basically differs in the point that the grip portion 30 is removed from the stratum corneum collecting device 10. That is, in the present embodiment, one end of the rod shaped portion 11 itself functions as a grip portion for gripping the stratum corneum collecting device 10.

Hereinafter, a method of use of the stratum corneum collecting/detecting kit 5 of the present embodiment will be described. In the case that the stratum corneum collecting/detecting kit 5 is employed as well, the collection of the stratum corneum using the stratum corneum collecting device 10 and the housing of the stratum corneum collecting device 10 in the solvent container 20 are basically conducted in the same manner as the stratum corneum collecting/detecting kit 3 of the third embodiment. However, in this case, the stratum corneum collecting device 10 is held by gripping one end of the rod shaped portion 11 as described above.

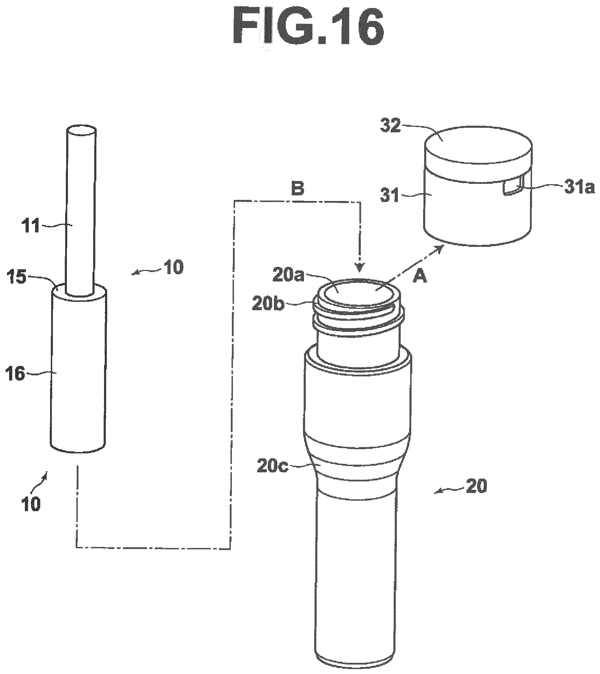

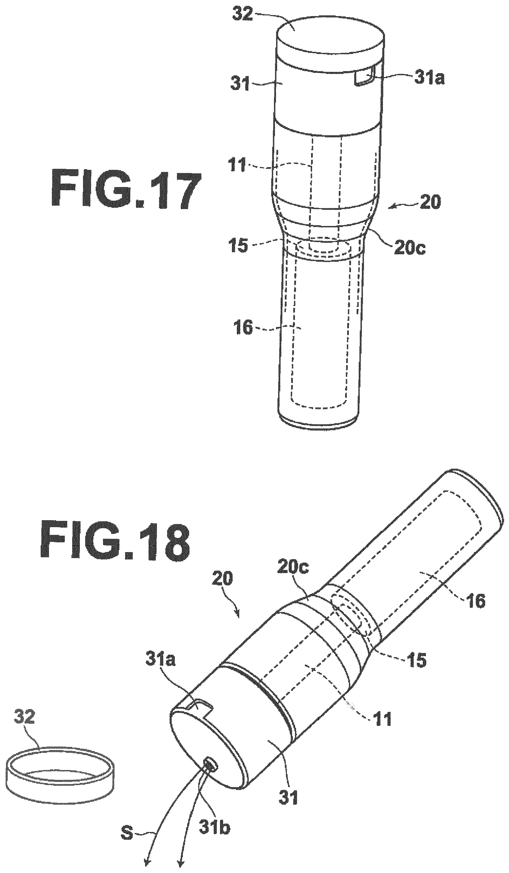

Prior to housing the stratum corneum collecting device 10 in the solvent container 20, the plug portion 31 is detached from the upper portion of the solvent container 20, as indicated by the dashed line A in Figure 16. The stratum corneum collecting device 10 is received in the solvent container 20 to be housed therein by the columnar portion 15 being inserted through the opening 20a, as indicated by the dashed line B in Figure 16. Thereafter, the plug portion 31 is attached to the upper portion of the solvent container 20 again, and the opening 20a is closed. The state of the stratum corneum collecting/detecting kit 5 at this time is illustrated in Figure 17. Note that a guide member (guide means) that loosely engages the rod shaped portion 11 is formed in the interior of the plug portion 31. This engagement causes the stratum corneum collecting device 10 to be held within the solvent container 20 coaxially with the solvent container 20 and in a state in which the adhesive layer 16 does not contact the inner wall of the solvent container 20.

In the manner described above, the adhesive layer 16 is immersed in the solvent within the solvent container 20 for a predetermined period of time to extract a specific component of the stratum corneum which has been collected and is held on the adhesive layer 16 into the solvent. Next, as illustrated in Figure 18, the engagement between the plug portion 31 and the cap 32 is released, and the cap 32 is removed from the plug portion 31. A solvent outlet 31b is open in the upper base portion of the plug portion 31. When the cap 32 is removed, the solvent outlet 31b is opened. In this state, when the solvent container 20 is brought into an orientation in which the upper portion thereof faces downward, the solvent S containing the extracted specific component of the stratum corneum is taken out from the solvent container 20 through the solvent outlet 31b.

### << Sixth Embodiment >>

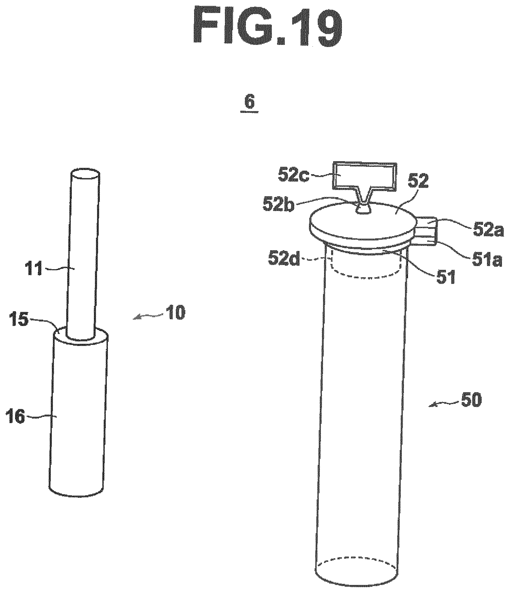

Next, a sixth embodiment of the present invention will be described with reference to Figures 19 through 23. As illustrated in Figure 19, the stratum corneum collecting/detecting kit 6 according to the sixth embodiment includes a stratum corneum collecting device 10 similar to that illustrated in Figure 15, and a relatively thin bottomed cylindrical solvent container 50.

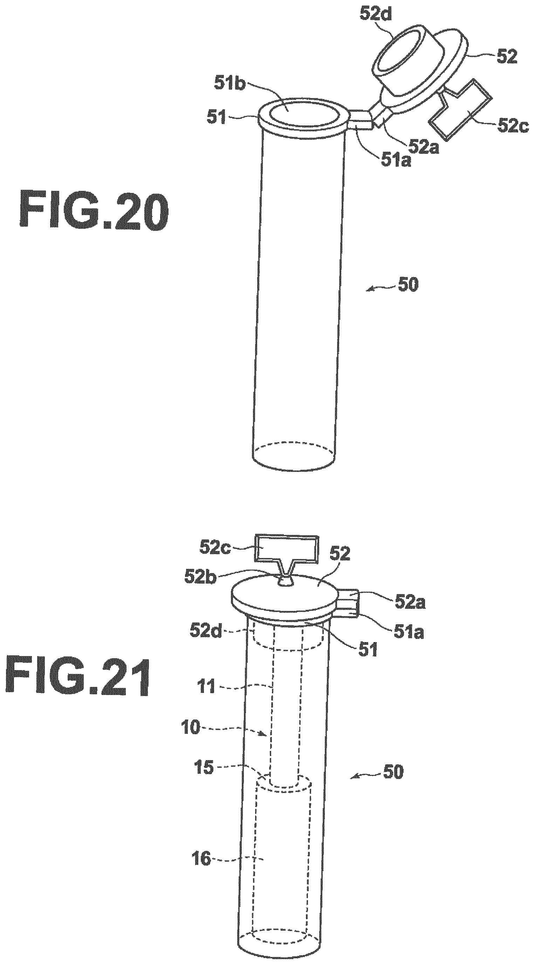

As also illustrated in Figure 20, the solvent container 50 has a flange portion 51 at the upper end thereof. The flange portion 51 has a hinge portion 51a which projects to the side and an opening 51b. The opening 51b of the flange portion 51 functions as an opening of the solvent container 50 for receiving the stratum corneum collecting device 10. Note that the solvent container 50 that includes the flange portion 51 and a lid 52 to be described later are formed by a synthetic resin, for example.

The lid 52 is disposed above the opening 51b. The lid 52 has a hinge portion 52a which projects to the side, and the hinge portion 52a and the hinge portion 51a achieve a hinge connection by a bend in the synthetic resin. Thereby, as illustrated in Figure 19, the lid 52 can assume a closed position in which the opening 51b is closed and an open position for in which the opening 51b is exposed, as illustrated in Figure 20.

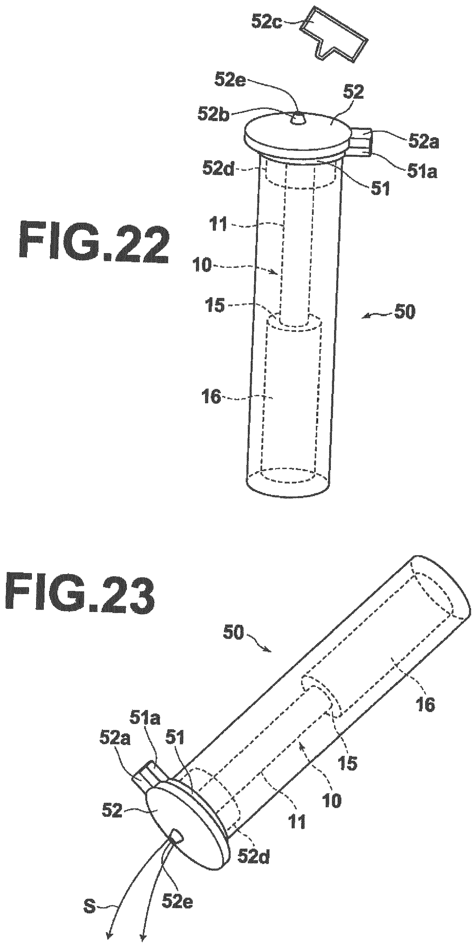

A solvent outflow portion 52b is formed on the upper surface of the lid 52. The solvent outflow portion 52b has a solvent outlet 52e (refer to Figures 22 and 23), which will be described later, and one end of the solvent outlet 52e is open at an inner surface of the lid 52 via a communicating aperture (not shown) formed in the lid 52. The other end of the solvent outlet 52e is closed by a peelable piece 52c which is integrally molded with the solvent outflow portion 52b prior to the stratum corneum collecting/detecting kit 6 being employed. A cylindrical portion 52d is formed on the inner surface of the lid 52, and when the lid 52 is in the closed position, the cylindrical portion 52d is fitted in the opening 51b substantially tightly.

Hereinafter, a method of use of the stratum corneum collecting/detecting kit 6 of the present embodiment will be described. In the case that the stratum corneum collecting/detecting kit 6 is employed as well, the collection of the stratum corneum using the stratum corneum collecting device 10 is conducted in the same manner as the stratum corneum collecting/detecting kit 5 illustrated in Figure 15.

The stratum corneum collecting device 10, having the stratum corneum collected and held on the adhesive layer 16, is received in the solvent container 50, in which the lid 52 is set in the open position, through the opening 51b as illustrated in Figure 20. When the stratum corneum collecting device 10 is housed in the solvent container 50, the lid 52 is set to the closed position and the opening 51b is closed. The state at this time is illustrated in Figure 21. In this manner, the adhesive layer 16, which has collected and is holding the stratum corneum, is immersed in the solvent in the solvent container 50. A guide member (guide means) that loosely fits the rod shaped portion 11 is formed in the interior of the cylindrical portion 52d. The fitting of the rod shaped portion 11 in the guide member causes the stratum corneum collecting device 10 to be held within the solvent container 50 coaxially with the solvent container 50 and in a state in which the adhesive layer 16 does not contact the inner wall of the solvent container 50.

After the adhesive layer 16 is immersed in the solvent for a predetermined amount of time, as illustrated in Figure 22, the peelable piece 52 c is peeled from the solvent outflow portion 52b. As a result, the solvent outlet 52e which is formed in the solvent outflow portion 52b is opened. The solvent outflow portion 52b is formed at a position outside the rod shaped portion 11 which is housed in the solvent container 50.

Then, as illustrated in Figure 23, when the solvent container 50 is brought into an orientation in which the upper part thereof faces downward, the solvent S containing the extracted specific component of the stratum corneum is taken out from the solvent container 50 through the solvent outlet 52e.

Note that in addition to the stratum corneum collecting device 10 being combined with the above-mentioned solvent container 20 or 50 to constitute a stratum corneum collecting/detection kit, the stratum corneum collecting device 10 may also be employed alone to collect stratum corneum. Using such a stratum corneum collecting device 10 enables stratum corneum to be collected with a simpler operation than that of the known tape stripping method and the like.

## Claims

1. A stratum corneum collecting/detecting kit, **characterized by** comprising:
a stratum corneum collecting device comprising a rod shaped portion having a grip portion at one end to be gripped by a user, a columnar portion which is continuous with the other end of the rod shaped portion, and an adhesive layer formed on a circumferential surface of the columnar portion;
a solvent container, which is a bottomed cylindrical container having an opening in an upper portion thereof, containing a solvent for extracting a component from stratum corneum collected by the adhesive layer, the solvent container receiving the columnar portion of the stratum corneum collecting device through the opening so as to enable the adhesive layer to be immersed in the solvent; and
a lid for closing the opening of the solvent container after the adhesive layer is immersed in the solvent.

2. A stratum corneum collecting/detecting kit as defined in Claim 1, wherein:
one of the solvent container and the lid is provided with a solvent outlet capable of maintaining a closed state, which is openable to take the solvent out from the interior to the exterior of the solvent container.

3. A stratum corneum collecting/detecting kit as defined in Claim 1 or Claim 2, wherein:
a cross sectional area of the internal space of the solvent container in a plane perpendicular to a cylinder axis direction is smaller at the portion that receives the columnar portion than at the portion where the opening is formed.

4. A stratum corneum collecting/detecting kit as defined in any one of Claims 1 through 3, wherein:
the amount of the solvent is set such that the liquid level of the solvent in the solvent container is located in the vicinity of the upper end of the adhesive layer which is immersed in the solvent.

5. A stratum corneum collecting/detecting kit as defined in any one of Claims 1 through 4, wherein:
the grip portion also functions as the lid.

6. A stratum corneum collecting/detecting kit as defined in any one of Claims 1 through 5, further comprising:
guide means for positioning the stratum corneum collecting device which is inserted in the solvent container such that the adhesive layer does not come in contact with an inner wall of the solvent container.

7. A stratum corneum collecting device, **characterized by** comprising:
a rod shaped portion having a grip portion at one end to be gripped by a user;
a columnar portion which is continuous with the other end of the rod shaped portion; and
an adhesive layer formed on a circumferential surface of the columnar portion.
